Europäisches Patentamt

European Patent Office

Office européen des brevets

(19)

(11) Veröffentlichungsnummer: **0 000 157**
**B1**

(12) **EUROPÄISCHE PATENTSCHRIFT**

(45) Veröffentlichungstag der Patentschrift: **01.04.81**

(21) Anmeldenummer: **78100175.5**

(22) Anmeldetag: **16.06.78**

(51) Int. Cl.³: **C 07 C 55/02,**
**C 08 G 69/28,**
**C 07 C 55/14, C 07 C 51/41**

(54) Verfahren zur Herstellung von hochkonzentrierten wässrigen Lösungen von Dicarbonsäurediaminsalzen und Polyamidpräkondensaten und deren Verwendung.

(30) Priorität: **27.06.77 DE 2728817**

(43) Veröffentlichungstag der Anmeldung:
**10.01.79 Patentblatt 79/1**

(45) Bekanntmachung des Hinweises auf die Patenterteilung:
**01.04.81 Patentblatt 81/13**

(84) Benannte Vertragsstaaten:
**BE DE FR GB NL**

(56) Entgegenhaltungen:
**GB - A - 543 843**
**GB - A - 898 339**
**US - A - 3 402 152**

(73) Patentinhaber: **BASF Aktiengesellschaft**
**Carl-Bosch-Strasse 38**
**D-6700 Ludwigshafen (DE)**

(72) Erfinder: **Eckell, Albrecht, Dr.**
**Paul-Klee-Strasse 2**
**D-6710 Frankenthal (DE)**
Erfinder: **Matthies, Paul, Dr.**
**Truebnerstrasse 59**
**D-6900 Heidelberg (DE)**
Erfinder: **Pilz, Georg**
**Ahornweg 4**
**D-6730 Neustadt 19 (DE)**
Erfinder: **Rotzoll, Rudi-Heinz, Dr.**
**Donnersbergstrasse 8**
**D-6703 Limburgerhof (DE)**

Courier Press, Leamington Spa, England.

## Verfahren zur Herstellung von hochkonzentrierten wäßrigen Lösungen von Dicarbonsäurediaminsalzen und Polyamidpräkondensaten und deren Verwendung

Gegenstand der Erfindung ist ein Verfahren zur Herstellung von 70 bis 90 gew.%igen, wäßrigen Lösungen von Salzen aus Dicarbonsäuren und Diaminen sowie Polyamidpräkondensaten durch Umsetzen von Alkandicarbonsäuren mit 6 bis 12 Kohlenstoffatomen und Alkandiaminen mit 6 bis 12 Kohlenstoffatomen in Wasser.

Bei der in der Technik wohl bekannten Herstellung von Polyamiden aus Dicarbonsäuren und Diaminen geht man in der Regel von wäßrigen Salzlösungen aus Dicarbonsäuren und Diaminen aus. Wie aus der GB—PS 674 954, der DT—PS 1 060 139 und der DT—AS 11 58 257 bekannt ist, geht man hierbei im allgemeinen von 45 bis 70 gewichtsprozentigen Salzlösungen aus. Vor der eigentlichen Polykondensation ist es jedoch erforderlich, aus den Salzlösungen das überschüssige Wasser zum großen Teil zu entfernen. Dies hat den Nachteil, daß man große Flüssigkeitsmengen bewegen muß und darüber hinaus erhebliche Mengen an Energie verbraucht, um das Wasser zu entfernen.

Entsprechend dem Verfahren nach der US—PS 3 402 152 werden wäßrige Lösungen von Salzen aus Dicarbonsäuren und Diaminen von z.B. 25 bis 55 Gew.% unter erhöhtem Druck bei 130° bis 165°C in einem Verdampfer auf einen Gehalt bis zu ca. 75 Gew.% gebracht. Hierbei treten immer noch Verluste an Diaminen bis zu 0,15 Gew.% auf, was unerwünscht ist.

Um überflüssige Mengen an Wasser zu vermeiden, wurde auch schon versucht, direkt von den flüssigen Ausgangsstoffen z.B. geschmolzener Adipinsäure und geschmolzenem Hexamethylendiamin bei der Polyamidherstellung auszugehen. Solche Verfahren sind beispielsweise aus der GB—PS 1 018 653 und BE—PS 640 369 bekannt. Abgesehen davon, daß sich geschmolzene Adipinsäure verändert, z.B. durch Decarboxylierung und Anhydridbildung, treten erhebliche Probleme bei der genauen Dosierung der Ausgangsstoffe auf. Dies gilt umso mehr als eine genaue Dosierung erforderlich ist, um äquivalente Verhältnisse einzuhalten.

Aus der DT—OS 24 03 178 ist auch schon bekannt, kristalline Salze aus Dicarbonsäuren und Diaminen herzustellen, indem man Dicarbonsäure enthaltende Salzlösungen mit dem jeweiligen Diamin neutralisiert. Hierbei sollten jedoch Temperaturen von 80°C nicht überschritten werden.

Es war die technische Aufgabe gestellt, Ausgangslösungen für die Herstellung von Polyamiden aus Dicarbonsäuren und Diaminen zur Verfügung zu stellen, die leicht handhabbar sind und möglichst wenig Wasser enthalten und die in den Ausgangslösungen enthaltene Wärmeenergie nutzbar zu machen.

Diese Aufgabe wird gelöst in einem Verfahren zur Herstellung von 70 bis 90 gew.%igen wäßrigen Lösungen von Salzen aus Dicarbonsäuren und Diaminen sowie Polyamidpräkondensaten durch Umsetzen von Alkandicarbonsäuren mit 6 bis 12 Kohlenstoffatomen und Alkandiaminen mit 6 bis 12 Kohlenstoffatomen in Wasser, dadurch gekennzeichnet, daß man 40 bis 65 gew.%ige wäßrige Salzlösungen aus Dicarbonsäuren und Diaminen von 60 bis 110°C, die eine entsprechende Menge der jeweiligen Dicarbonsäure in Überschuß gelöst enthalten, mit dem jeweiligen geschmolzenen Diamin in äquivalenten Mengen bezogen auf gelöste Dicarbonsäure in an sich bekannter Weise umsetzt, mit der Maßgabe, daß die Umsetzung unter erhöhtem Druck durchgeführt wird und eine Endtemperatur von 160 bis 200°C bei der Umsetzung eingehalten wird.

Das neue Verfahren hat den Vorteil, daß man unmittelbar zu hochkonzentrierten wäßrigen Ausgangslösungen für die Polyamidherstellung gelangt, die nicht mehr weiter konzentriert werden müssen. Weiter hat das neue Verfahren den Vorteil, daß man Ausgangslösungen erhält, in denen die enthaltene Neutralisationswärme für die Polykondensation genutzt wird. Schließlich hat das neue Verfahren den Vorteil, daß die Dosierung der Ausgangsstoffe und deren Vermischung einfach zu bewerkstelligen ist.

Im Hinblick auf die DT—OS 2 403 178 war es nicht angezeigt, bei der Neutralisation von Dicarbonsäuren mit Diaminen Temperaturen von 80°C zu überschreiten, da bei höheren Temperaturen für die Polykondensation unbrauchbare Produkte zu erwarten waren.

Erfindungsgemäß geht man von Alkandicarbonsäuren mit 6 bis 12 Kohlenstoffatomen aus. Bevorzugt verwendet man geradkettige $\alpha$-$\omega$-Dicarbonsäuren der genannten Kohlenstoffzahl. Geeignete Dicarbonsäuren sind beispielsweise Adipinsäure, Korksäure, Azelainsäure, Sebacinsäure, Dekandisäure und Dodecandisäure. Besondere technische Bedeutung haben Adipinsäure und Sebacinsäure erlangt.

Ferner geht man von Alkandiaminen mit 6 bis 12 Kohlenstoffatomen aus. Besonders bevorzugt verwendet man geradkettige $\alpha$-$\omega$-Alkandiamine mit 6 bis 12 Kohlenstoffatomen. Geeignete Diamine sind beispielsweise Hexamethylendiamin. Octamethylendiamin, Decamethylendiamin oder Dodecamethylendiamin.

Besondere technische Bedeutung hat Hexamethylendiamin erlangt. Demzufolge sind auch Lösungen von Salzen und Polyamidpräkondensaten, die sich von Adipinsäure oder Sebacinsäure und Hexamethylendiamin ableiten, bevorzugte Verfahrensprodukte.

Man geht zunächst von einer wäßrigen Salzlösung aus Dicarbonsäuren und Diaminen von 40 bis 65 Gewichtsprozent aus, die eine entsprechende Menge der jeweiligen Dicarbonsäure im Überschuß gelöst enthält. Vorteilhaft verwendet man Lösungen, die 50 bis 65 Gewichtsprozent an den jeweiligen Salzen, bezogen auf die Summe von Salz und Wasser, enthalten. Die Menge an gelöster Dicarbonsäure richtet sich nach der gewünschten Endkonzentration der herzustellenden hochkonzentrierten wäßrigen Lösungen.

Nach einer bevorzugten Ausführungsform löst man in einer Salzlösung geringerer Konzentration feste Dicarbonsäure auf. Hierbei hält man Temperaturen von 60 bis 110°C ein. So erhaltene freie Dicarbonsäure enthaltende Salzlösung wird dann mit geschmolzenem Diamin in äquivalenten Mengen bezogen auf die gelöste Dicarbonsäure umgesetzt. Vorteilhaft achtet man darauf, daß man den pH-Wert für den Äquivalenzpunkt des jeweils herzustellenden Salzes einhält. Beispielsweise ist der Äquivalenzpunkt für Hexamethylendiammoniumadipat pH 7,62 und für Hexamethylendiammoniumsebacat pH 7,5 gemessen in 10 prozentiger wäßriger Lösung bei 25°C. Vorteilhaft führt man die Umsetzung in einer Mischstrecke durch, wobei man zunächst einen geringen Unterschuß an geschmolzenem Diamin zugibt und dann die präzise Einstellung durch eine weitere Zugabe vom geschmolzenen Diamin durchführt. Um den bei der Polykondensation eintretenden Verlust an Diaminen auszugleichen, verwendet man Diamine in der Regel im Überschuß z.B. 1,5 Molprozent.

Nach einer anderen bevorzugten Ausführungsform löst man zunächst feste Dicarbonsäure in einer wäßrigen Lösung von Diamin im Unterschuß. Hierbei erhält man eine Salzlösung aus Dicarbonsäure und Diamin, die eine überschüssige Menge an Dicarbonsäure gelöst enthält. Es versteht sich, daß man die Mengenverhältnisse so wählt, daß man eine Salzlösung der vorgenannten Konzentration erhält und die überschüssige Menge an Dicarbonsäure der gewünschten Endkonzentration der wäßrigen Lösung entspricht. Das Lösen der Dicarbonsäure erfolgt bei Temperaturen von 60 bis 110°C, wobei man in der Regel atmosphärischen Druck einhält. Die so erhaltene freie Dicarbonsäure enthaltende Salzlösung wird dann mit geschmolzenem Diamin in äquivalenten Mengen bezogen auf gelöste Dicarbonsäure umgesetzt. Diese Umsetzung erfolgt vorteilhaft wie bei der vorgenannten Arbeitsweise beschrieben.

Es ist ein wesentliches Merkmal der Erfindung, daß die Umsetzung der gelösten Dicarbonsäure mit geschmolzenem Diamin unter erhöhtem Druck durchgeführt wird. Vorteilhaft erhält man Drücke von 2 bis 15 bar ein. Ein entsprechender Druck stellt sich durch den autogenen Druck von selbst ein und kann noch durch geringe Stickstoffzugaben erhöht werden, um ein Sieden der Lösung zu verhindern. Ferner ist es ein wesentliches Merkmal der Erfindung, daß man bei der Neutralisation eine Endtemperatur von 160 bis 200°C einhält.

So erhaltene wäßrige Lösungen enthalten Salze aus Dicarbonsäuren und Diaminen. Sie können durch beginnende Polykondensation auch Polyamidpräkondensate enthalten. Der Umsatz der Amino- und Carboxylgruppen kann bis zu 50% z.B. 20 bis 45% betragen.

Die erzeugten wäßrigen Lösungen haben einen Gehalt von 70 bis 90 Gewichtsprozent, insbesondere 80 bis 90 Gewichtsprozent an Salzen aus Dicarbonsäuren und Diaminen und Polyamidpräkondensaten.

Erfindungsgemäß hergestellte hochkonzentrierte wäßrige Lösungen von Salzen und Polyamidpräkondensaten, die sich von Dicarbonsäuren und Diaminen ableiten eignen sich in hervorragender Weise zur Herstellung von Polyamiden durch Kondensation. Die Kondensation kann absatzweise oder kontinuierlich durchgeführt werden. Geeignete Kondensationsverfahren werden beispielsweise beschrieben in der DT—AS 14 95 087, GB—PS 1 159 151, GB—PS 674 954 oder DT—PS 1 060 139 und DT—OS 24 17 003. Besonders vorteilhaft ist es, die erfindungsgemäßen, wäßrigen Lösungen für die Kondensation zu verwenden, ohne vorher die Neutralisationswärme abzuführen. Unbeachtlich sind in diesem Zusammenhang apparativ bedingte Wärmeverluste.

Es ist auch möglich, einen Teil der Neutralisationswärme zum Lösen der Dicarbonsäuren in den Salzlösungen geringerer Konzentration zu verwenden.

Polyamide, die aus den erfindungsgemäßen Lösungen hergestellt werden, eignen sich zur Erzeugung von aus der Schmelze geformten Massen, wie Fäden, Fasern, Formteile, Platten oder Überzügen.

Das Verfahren nach der Erfindung sei in folgenden Beispielen veranschaulicht.

Beispiel 1

5250 g AH-Salz (Hexamethylendiammoniumadipat) wurden in einem 40-l-Rührautoklaven in 3450 g Wasser unter Erwärmen auf 95°C gelöst. Zu der so erhaltenen 60,3 prozentigen AH-Salz-Lösung wurden 8025 g feste Adipinsäure allmählich unter Rühren bei 90 bis 95°C zugegeben und gelöst. Der Autoklav wurde mit Stickstoff gespült und verschlossen. Der Inhalt wurde auf 100°C erhitzt, wobei sich ein Druck von 2 bar einstellte (alle Druckangaben sind Absolutwerte). 6375 g geschmolzenes Hexamethylendiamin von 100°C wurde aus einem Zulaufgefäß mit Stickstoffdruck innerhalb von 2 Minuten bei laufendem Rührer in den Autoklaven gedrückt. In der so erhaltenen 85 gewichtsprozentigen AH-Salz-Lösung stieg die Temperatur durch die freiwerdende Neutralisationswärme an und erreichte 2 Minuten nach Zugabe des Hexamethylendiamins 162°C. Während der Zugabe des Hexamethylendiamins stieg der Druck vorübergehend auf 4 bar und fiel bis zum Ende der Zugabe auf 3 bar.

In analoger Weise erhält man Nylon® 6,9 und Nylon® 10,6 Salzlösungen.

### Beispiel 2

Das Beispiel zeigt die Ausnutzung eines Teils der Neutralisationswärme zum Lösen der Dicarbonsäure.

In einem Glaskolben mit Rührer, Rückflußkühler, Thermometer und Tropftrichter wurden 292 g feste Adipinsäure bei 20°C vorgelegt.

Der Kolben wurde in ein als Schutzheizung wirkendes Wasserbad von 75°C eingetaucht; sofort danach wurde eine im Tropftrichter auf 90°C gehaltene Lösung von 75 g Hexamethylendiamin in 92 g Wasser innerhalb von 20 Sekunden unter Rühren zugegeben. Nach 2 Minuten war eine klare Lösung entstanden, deren Temperatur 69°C betrug. Die Zusammensetzung der Lösung war 37% AH-Salz, 43% Adipinsäure und 20% Wasser. Die weitere Umsetzung erfolgte analog Beispiel 1.

### Beispiel 3

Die im Beispiel 1 erhaltene AH-Salz-Lösung wurde ohne Abkühlen der Lösung polykondensiert. Sie wurde innerhalb von 3 1/2 Stunden auf 275°C erhitzt, wobei der Druck durch Abblasen von Wasserdampf auf 19 bar gehalten wurde. Nach Erreichen von 275°C wurde innerhalb von 1 Stunde auf Normaldruck entspannt und 1 Stunde bis 275°C nachkondensiert. Das Produkt wurde mit Stickstoffdruck aus dem Autoklaven herausgedruckt, wobei der Schmelzstrang in einem Wasserbad abgekühlt und granuliert wurde. Das erhaltene Polyamid 66 hatte eine relative Viskosität von 2,54, gemessen in 1 prozentiger Lösung in 96 prozentiger Schwefelsäure, und einen Gehalt an sauren Gruppen von 54 mÄq/kg und an basischen Gruppen von 76 mÄq/kg.

### Patentansprüche

1. Verfahren zur Herstellung von 70 bis 90 gew.-%igen wäßrigen Lösungen von Salzen aus Dicarbonsäuren und Diaminen sowie Polyamidpräkondensaten durch Umsetzen von Alkandicarbonsäuren mit 6 bis 12 Kohlenstoffatomen und Alkandiaminen mit 6 bis 12 Kohlenstoffatomen in Wasser, dadurch gekennzeichnet, daß man 40 bis 65 gew.-%ige wäßrige Salzlösungen aus Dicarbonsäuren und Diaminen von 60 bis 110°C, die eine entsprechende Menge der jeweiligen Dicarbonsäure im Überschuß gelöst enthalten mit dem jeweiligen geschmolzenen Diamin in äquivalenten Mengen bezogen auf die gelöste Dicarbonsäure in an sich bekannter Weise umsetzt, mit der Maßgabe, daß die Umsetzung unter erhöhtem Druck durchgeführt wird und eine Endtemperatur von 160 bis 200°C bei der Umsetzung eingehalten wird.

2. Verfahren nach Anspruch 1, dadurch gekennzeichnet, daß man einen Druck von 2 bis 15 bar einhält.

3. Verfahren nach den Ansprüchen 1 und 2, dadurch gekennzeichnet, daß man Dicarbonsäure in einer wäßrigen Salzlösung aus Dicarbonsäuren und Diaminen löst und mit geschmolzenem Diamin umsetzt.

4. Verfahren nach den Ansprüchen 1 und 2, dadurch gekennzeichnet, daß man feste Dicarbonsäure mit wäßrigem Diamin im Unterschuß mischt und die so erhaltene Salzlösung geringerer Konzentration die Dicarbonsäure gelöst enthält, mit geschmolzenem Diamin umsetzt.

5. Verfahren nach den Ansprüchen 1 bis 4, dadurch gekennzeichnet, daß man einen Teil der Neutralisationswärme zum Lösen der Dicarbonsäure benutzt.

6. Verwendung von 70 bis 90 gew.-%igen wäßrigen Lösungen von Salzen und Polyamidpräkondensaten gemäß Anspruch 1 zur Herstellung von Polyamiden durch Kondensation.

7. Verwendung von hochkonzentrierten wäßrigen Lösungen von Salzen und Polyamidpräkondensaten nach Anspruch 6, dadurch gekennzeichnet, daß die Lösungen ohne vorherige Abführung der Neutralisationswärme der Kondensation zugeführt werden.

### Claims

1. A process for the manufacture of a 70 to 90% by weight aqueous solution of a salt of a dicarboxylic acid and a diamine, as well as of a nylon precondensate by reacting an alkanedicarboxylic acid of 6 to 12 carbon atoms and an alkanediamine of 6 to 12 carbon atoms, characterized in that a 40 to 65% by weight aqueous solution of a salt of a dicarboxylic acid and a diamine, containing an appropriate dissolved excess of the particular dicarboxylic acid, is reacted in a conventional manner with the particular diamine in the molten state, in an equivalent amount to the dissolved dicarboxylic acid, with the proviso that the reaction is carried out under superatmospheric pressure and the final reaction temperature is kept at from 160 to 200°C.

2. A process as claimed in claim 1, characterized in that the pressure is maintained at from 2 to 15 bars.

3. A process as claimed in claims 1 and 2, characterized in that a dicarboxylic acid is dissolved in an aqueous solution of a salt of a dicarboxylic acid with a diamine, and is then reacted with molten diamine.

4. A process as claimed in claims 1 and 2, characterized in that a solid dicarboxylic acid is mixed with a less than equivalent amount of aqueous diamine and the resulting salt solution of lower concentration, which contains dissolved dicarboxylic acid, is reacted with molten diamine.

5. A process as claimed in claims 1 to 4, characterized in that a part of the heat of neutralization is utilized to dissolve the dicarboxylic acid.

6. Use of a 70 to 90% by weight aqueous solution of a salt as well as of a nylon precondensate according to claim 1 for the manufacture of a nylon by condensation.

7. Use of a highly concentrated aqueous solution of a salt as well as of a nylon precondensate as claimed in claim 6, characterized in that the solution is fed to the condensation without prior removal of the heat of neutralization.

## Revendications

1. Procédé de préparation de solutions aqueuses, à 70 à 90% en poids, de sels d'acides dicarboxyliques et de diamines, ainsi que de précondensats de polyamides, par réaction dans l'eau d'acides alcanedicarboxyliques de 6 à 12 atomes de carbone et d'alcanediamines de 6 à 12 atomes de carbone, caractérisé par le fait que l'on fait réagir, de manière connue en soi, des solutions de sels, aqueuses, à 40 à 65% en poids, d'acides dicarboxyliques et de diamines, entre 60 et 110°C, qui contiennent, dissoute en excès, une quantité correspondante des acides dicarboxyliques correspondants, avec la diamine fondue correspondante en proportions équivalentes, rapportées aux acides dicarboxyliques dissous, en prenant soin que la réaction soit effectuée sous pression élevée et que, lors de la réaction, soit respectée une température finale de 160 à 200°C.

2. Procédé selon la revendication 1, dans lequel on respecte une pression de 2 à 15 bars.

3. Procédé selon l'une des revendications 1 et 2, dans lequel on dissout de l'acide dicarboxylique dans une solution de sels aqueuse d'acides dicarboxyliques et de diamines et on fait réagir avec de la diamine fondue.

4. Procédé selon l'une des revendications 1 et 2, dans lequel on mélange des acides dicarboxyliques solides avec de la diamine aqueuse en quantité insuffisante et on fait réagir avec de la diamine fondue la solution de sels ainsi obtenue, de concentration inférieure, qui contient de l'acide dicarboxylique dissous.

5. Procédé selon l'une des revendications 1 à 4, dans lequel on utilise une partie de la chaleur de neutralisation pour dissoudre l'acide dicarboxylique.

6. Utilisation de solutions aqueuses à 70 à 90% en poids, de sels et de précondensats de polyamides selon la revendication 1 pour préparer des polyamides par condensation.

7. Utilisation de solutions aqueuses à forte concentration de sels et de précondensats de polyamides selon la revendication 6, caractérisée par le fait que l'on amène à la condensation les solutions sans évacuation préalable de la chaleur de neutralisation.